# EUROPEAN PATENT SPECIFICATION

(11) **EP 0 720 606 B1**
(45) Date of publication and mention of the grant of the patent: **07.05.1997**
(21) Application number: 94928382.4
(22) Date of filing: 19.09.1994
(51) Int. Cl.: C07D 301/10

(54) **ARGON REMOVAL IN A PROCESS FOR ETHYLENE OXIDE PRODUCTION**
ENTFERNUNG VON ARGON IN EINEM VERFAHREN ZUR HERSTELLUNG VON ETHYLENOXID
ENLEVEMENT DE L'ARGON DANS UN PROCEDE DE PRODUCTION D'OXYDE D'ETHYLENE

(30) Priority: 20.09.1993 EP 93202726
(43) Date of publication of application: 10.07.1996
(73) Proprietor: SHELL INTERNATIONALE RESEARCH MAATSCHAPPIJ B.V., 2596 HR Den Haag (NL)
(72) Inventor: GORCESTER, Jeffrey, Paul c/o Shell UK Expl & Prod., UK-Aberdeen AB9 2HY (GB)
(86) International application number: PCT/EP94/03144
(87) International publication number: WO 95/08545

(56) References cited:
- EP-A- 0 142 005
- EP-A- 0 200 518
- EP-A- 0 266 271
- EP-A- 0 481 363

## Description

The present invention relates to a process of manufacturing ethylene oxide.

European patent application publication No. 200 518 discloses a process of manufacturing ethylene oxide comprising allowing to react in a reactor ethylene and free oxygen to ethylene oxide in the presence of methane, carbon dioxide and argon; withdrawing from the reactor a reactor effluent; removing ethylene oxide from the reactor effluent to obtain a recycle stream; removing carbon dioxide and argon from the recycle stream to obtain a treated recycle stream; supplying the treated recycle stream to the reactor; and supplying additional reactants and methane to the reactor.

Additional reactants, ethylene and free oxygen-containing gas, are supplied to the reactor to replace the reactants which reacted to ethylene oxide. Methane is supplied to maintain the methane concentration at such a level that its presence is beneficial to the efficiency of the reaction as described in USA patent specification No. 3 119 837.

Carbon dioxide is a by-product of the reaction and part of the carbon dioxide is removed to maintain the concentration of carbon dioxide below a predetermined level.

Argon enters into the process with the free oxygen-containing gas. In order to maintain the argon concentration below a predetermined level, part of this contaminant is removed from the recycle stream downstream of the removal of ethylene oxide. In addition trace amounts of nitrogen enter into the process with the free oxygen-containing gas.

In the known process removing argon comprises separating a side stream from the recycle stream, passing the side stream to the feed inlet of a membrane unit, removing an argon-containing stream from the permeate outlet of the membrane unit, removing a retentate stream from the retentate outlet of the membrane unit, and adding the retentate stream to the recycle stream.

As the flow through the membrane is large, the area of the membrane is large as well.

It is an object of the present invention to reduce the area of the membrane in the membrane unit.

To this end the process of manufacturing ethylene oxide according to the present invention is characterized in that the permeate side of the membrane unit is purged with methane.

As such purging the permeate side of a membrane unit is known. European patent application publication No. 481 363 relates to improving a membrane process for drying air using a membrane unit wherein the permeate side of the membrane unit is purged with dry gas, wherein the separation factor water/air is much greater than the separation factor of argon/ethylene. European patent application publication No. 266 271 also relates to improving the separation gases in a membrane separation process wherein the permeate side is purged, the improvement being that the partial pressures of certain gaseous components in the purge gas are so that substantially eliminated. However, this publication is not relevant as the component to be retained is used to purge the permeate side.

Reference is further made to European patent application publication No. 142 005. This publication relates to an ammonia synthesis process, and in particular to recovering hydrogen from a gas mixture and recycling the recovered hydrogen to the synthesis process. The hydrogen is recovered in a membrane unit, wherein permeate side of the membrane unit is purged and the permeated hydrogen is recycled to the process with the purge gas. In contrast to the present case this publication teaches to return the permeate to the synthesis process and not the retentate.

These publications are not relevant to the present invention as they do not disclose to apply purging the permeate side of a membrane unit in a membrane unit for removing argon with methane.

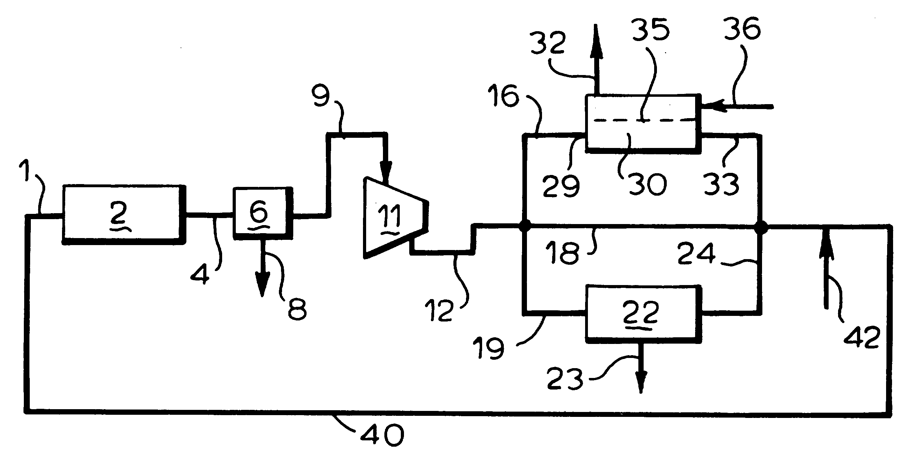

The invention will now be described by way of example in more detail with reference to the enclosed Figure showing schematically a line-up of the process of the present invention.

A mixture including ethylene, free oxygen-containing gas, argon, carbon dioxide and methane is supplied through conduit 1 to a reactor 2. In the reactor 2 ethylene and free oxygen are allowed to react to ethylene oxide in the presence of methane and contaminants such as carbon dioxide and argon. The reactor 2 contains a suitable catalyst. The catalyst, the reaction conditions and the concentration of the reactants are known per se, and as they are not essential to the present invention they will not be discussed.

From the reactor 2 a reactor effluent is withdrawn through conduit 4. The reactor effluent includes ethylene oxide, carbon dioxide, methane and argon. Carbon dioxide is a by-product of the reaction, a further by-product is ethane. Argon enters into the process with the free oxygen-containing gas, in addition trace amounts of nitrogen enter into the process with the free oxygen-containing gas.

Ethylene oxide is removed from the reactor effluent in an ethylene oxide removal unit 6, and from the ethylene oxide removal unit 6 are removed an ethylene oxide-containing stream through conduit 8 and a recycle stream through conduit 9. The ethylene oxide removal unit is known per se and will not be discussed.

The other contaminants are removed downstream of the ethylene oxide removal unit. For the present invention we are not concerned with the removal of nitrogen and ethane.

Downstream of the ethylene oxide removal unit 6 the recycle stream is compressed in compressor 11 to compensate for the pressure drops in the treating units.

The compressed recycle stream in conduit 12 is divided into a side stream 16 and a main stream. The main stream is divided into two streams 18 and 19. Carbon dioxide is removed from stream 19 in carbon dioxide removal unit 22. From the carbon dioxide removal unit 22 are withdrawn a carbon dioxide-containing stream through conduit 23 and a treated stream having a reduced carbon dioxide content through conduit 24. The carbon dioxide removal unit is known per se and will not be discussed. The flow rate of the side stream 16 is between 1 and 5% of the flow rate of the recycle stream, the flow rate of stream 19 is between 10 and 40% of the flow rate of the recycle stream, and the low rate of stream 18 is the balance.

Removing argon from the recycle stream comprises passing the side stream 16 to the feed inlet 29 of a membrane unit 30, removing an argon-containing stream from the permeate outlet 32, and removing a treated stream from the retentate outlet 33. The membrane unit 30 comprises a hollow fibre membrane schematically represented by dashed line 35, the hollow fibres include polysulfone. To purge the permeate side of the membrane unit 30, methane is supplied to the membrane unit 30 through conduit 36, the methane flows counter-currently through the permeate side of the membrane unit 30.

The treated stream 24 from the carbon dioxide removal unit 22 and the treated stream 33 from the membrane unit 30 are combined with the untreated stream 18 and supplied through conduit 40 and conduit 1 into the reactor 2. Additional reactants, ethylene and free oxygen-containing gas, and methane are supplied to the reactor 2 through conduit 42 which opens into conduit 40.

To illustrate the effect of purging the performance of the membrane unit 30 in case the permeate side is not purged is compared with the performance in case the permeate side is purged.

Table 1 gives an example of the compositions and the flow rates of the components in the feed to a membrane unit used in the process without purging, and in the retentate and the permeate leaving the membrane unit. The membrane unit includes a hollow fibre membrane having an area of 1 630 m², the hollow fibres include polysulfone.

Table 1. Compositions and flow rates of the feed, permeate and retentate of the membrane unit without purging (not according to the present invention).

| | Feed | | Permeate | | Retentate | |
|---|---|---|---|---|---|---|
| | %vol | kmol/h | %vol | kmol/h | %vol | kmol/h |
| Nitrogen | 1.22 | 4.54 | 0.81 | 0.14 | 1.24 | 4.40 |
| Argon | 9.30 | 34.60 | 11.88 | 2.09 | 9.17 | 32.51 |
| Oxygen | 4.16 | 15.48 | 7.70 | 1.36 | 3.98 | 14.12 |
| Methane | 50.24 | 186.90 | 38.36 | 6.75 | 50.83 | 180.15 |
| Ethylene | 27.20 | 101.19 | 22.72 | 4.00 | 27.42 | 97.19 |
| Ethane | 0.95 | 3.53 | 0.63 | 0.11 | 0.97 | 3.42 |
| Carbon dioxide | 6.93 | 25.78 | 17.90 | 3.15 | 6.39 | 22.63 |
| total | 100.00 | 372.02 | 100.00 | 17.60 | 100.00 | 354.42 |

Table 2 gives the compositions and the flow rates of the components in the feed to a membrane unit used in the process of the present invention, and in the retentate and the permeate leaving the membrane unit. The membrane unit includes a hollow fibre membrane having an area of 1 310 m², the hollow fibres include polysulfone. The purge gas is methane and the flow rate of the purge gas is 7.44 kmol methane per hour, which corresponds to 2 mol% of the feed flow rate.

Table 2. Compositions and flow rates of the feed, permeate and retentate of the membrane unit with purging (according to the present invention).

| | Feed | | Permeate | | Retentate | |
|---|---|---|---|---|---|---|
| | %vol | kmol/h | %vol | kmol/h | %vol | kmol/h |
| Nitrogen | 1.34 | 4.99 | 0.59 | 0.14 | 1.36 | 4.85 |
| Argon | 9.28 | 34.52 | 8.85 | 2.09 | 9.11 | 32.44 |
| Oxygen | 4.15 | 15.44 | 7.19 | 1.70 | 3.86 | 13.74 |
| Methane | 50.12 | 186.46 | 50.09 | 11.82 | 51.17 | 174.64 |
| Ethylene | 27.14 | 100.97 | 15.54 | 3.67 | 27.35 | 97.30 |
| Ethane | 1.06 | 3.94 | 0.47 | 0.11 | 1.08 | 3.83 |
| Carbon dioxide | 6.91 | 25.71 | 17.28 | 4.08 | 6.07 | 21.63 |
| total | 100.00 | 372.02 | 100.01 | 23.60 | 100.00 | 348.42 |

Comparing the performances of the two membrane units, it is clear that with purging the same amount of argon passes through a membrane with a significantly smaller area, and that a smaller amount of ethylene passes through the membrane.

The flow rate of the purge gas is suitably in the range of from 1 to 10 mol% of the feed flow rate. The amount of ethylene permeating through the membrane decreases with increasing purge gas flow rate, however, above 10 mol% the decrease is too small to justify such a large purge gas flow rate.

In the process described with reference to the Figure the main stream is divided into streams 18 and 19, and carbon dioxide is removed from stream 19. Alternatively, carbon dioxide can be removed from the main stream, so that dividing the main stream can be omitted.

The membrane material can be any material that allows selective permeation of argon. Suitably the membrane is a dense membrane of a polymeric material such as polydimethyl siloxane or polysulfone.

## Claims

1. A process of manufacturing ethylene oxide comprising allowing to react in a reactor (2) ethylene and free oxygen to ethylene oxide in the presence of methane, carbon dioxide and argon; withdrawing from the reactor (2) a reactor effluent (4); removing ethylene oxide from the reactor effluent to obtain a recycle stream (9); removing carbon dioxide (22) and argon (30) from the recycle stream to obtain a treated recycle stream (40); supplying the treated recycle stream (40) to the reactor(2); and supplying (42) additional reactants and methane to the reactor, wherein removing argon comprises separating a side stream (16) from the recycle stream, passing the side stream (16) to the feed inlet (29) of a membrane unit (30), removing an argon-containing stream (32) from the permeate outlet of the membrane unit (30), removing a retentate stream (33) from the retentate outlet of the membrane unit (30), and adding the retentate stream to the recycle stream,
characterized in that the permeate side of the membrane unit (30) is purged with methane (36).

2. Process as claimed in claim 2, wherein to purge the permeate side of the membrane unit (30), the methane purge gas (36) is passed counter-currently along the membrane (35).

3. Process as claimed in claim 1 or 2, wherein the flow rate of the methane purge gas (36) is in the range of from 1 to 10 mol% of the flow rate of the feed (16).

## Patentansprüche

1. Verfahren zur Herstellung von Ethylenoxid, bei dem man in einem Reaktor (2) Ethylen und freien Sauerstoff in Gegenwart von Methan, Kohlendioxid und Argon zu Ethylenoxid umsetzt, aus dem Reaktor (2) einen Reaktoraustrag (4) abzieht, aus dem Reaktoraustag Ethylenoxid abtrennt, wobei man einen Rückführstom (9) erhält, aus dem Rückführstrom Kohlendioxid (22) und Argon (30) entfernt, wobei man einen behandelten Rückführstrom (40) erhält, den behandelten Rückführstrom (40) dem Reaktor (2) zuführt und dem Reaktor zusätzliche Reaktanden und Methan zuführt (42), wobei man das Argon entfernt, indem man aus dem Rückführstrom einen Teilstrom (16) abzweigt, den Teilstrom (16) dem Einlaß (29) einer Membrananlage (30) zuführt, vom Permeat-Auslaß der Membrananlage (30) einen argonhaltigen Strom (32) abtrennt, vom Retentat-Auslaß der Membrananlage (30) einen Retentatstrom (33) abzieht und den Retentatstrom dem Rückführstrom zusetzt,
dadurch gekennzeichnet, daß man die Permeatseite der Membrananlage (30) mit Methan (36) spült.

2. Verfahren nach Anspruch 2, bei dem man zum Spülen der Permeatseite der Membrananlage (30) das Methanspülgas (36) im Gegenstrom an der Membran (35) entlangführt.

3. Verfahren nach Anspruch 1 oder 2, bei dem man die Strömungsrate des Methanspülgases (36) in einem Bereich von 1 bis 10 Mol-% der Strömungsrate des Einsatzstoffs (16) einstellt.

## Revendications

1. Procédé de fabrication de l'oxyde d'éthylène, conformément auquel on fait réagir dans un réacteur (2), de l'éthylène et de l'oxygène libre pour donner de l'oxyde d'éthylène en présence de méthane, de dioxyde de carbone et d'argon; on prélève du réacteur (2) un effluent du réacteur (4); on enlève de l'oxyde d'éthylène de l'effluent du réacteur pour obtenir un courant de recyclage (9); on enlève du dioxyde de carbone (22) et de l'argon (30) du courant de recyclage pour obtenir un courant de recyclage traité (40); on envoie le courant de recyclage traité (40) dans le réacteur (2) et on envoie (42) des réactifs supplémentaires et du méthane dans le réacteur, où l'enlèvement de l'argon comprend la séparation d'un courant latéral (16) du courant de recyclage, le passage du courant de recyclage (16) dans l'entrée (29) de l'alimentation d'une unité à membrane (30), l'enlèvement d'un courant contenant de l'argon (32) de la sortie du perméat de l'unité à membrane (30), l'enlèvement d'un courant de rétentat (33) de la sortie du rétentat de l'unité à membrane (30) et l'addition du courant de rétentat au courant de recyclage,
caractérisé en ce que l'on purge le côté perméat de l'unité à membrane (30) avec du méthane (36).

2. Procédé suivant la revendication 2, caractérisé en ce que pour purger le côté perméat de l'unité à membrane (30), on fait passer le gaz de purge qu'est le méthane (36) en contre-courant le long de la membrane (35).

3. Procédé suivant la revendication 1 ou 2, caractérisé en ce que la vitesse d'écoulement du gaz de purge qu'est le méthane (36) varie de 1 à 10% molaires de la vitesse d'écoulement de l'alimentation (16).
